# EUROPEAN PATENT APPLICATION

(11) **EP 4 317 397 A1**
(43) Date of publication of application: **07.02.2024**
(21) Application number: 22188334.1
(22) Date of filing: 02.08.2022
(51) Int. Cl.: C12M 1/00, C12M 1/12

(54) **MULTI-WELL PLATE SYSTEM FOR ASSESSING CELL LAYERS**

(71) Applicant: Simplinext SA, 2504 Bienne (CH)
(72) Inventor: Angeloni Suter, Silvia, 2072 Saint-Blaise (CH); Suter, Kaspar, 2072 Saint-Blaise (CH)
(74) Representative: P&TS SA (AG, Ltd.)

(57) **Abstract**

The invention concerns a well insert (1) for a multi-well plate (2) and a multi-well plate system for use in cell culture. The well insert comprises a planar support (14) having at least one permeable or semi-permeable portion (11), at least one pair of electrodes having a first electrode (41) and a second electrode (42) arranged to measure electrical impedance across a layered cell culture supported by the planar support (14), an insert wall with a projecting portion (13), said projecting portion being configured to interface with the multi-well plate (2) such as to suspend the insert (1) in a well (20), wherein the insert wall (12) together with the planar support (14) separate a first chamber (8) from a second chamber (9), the electrodes (41, 42) being arranged along the inner surface (15) of the insert and the counter-electrode (41, 42) being arranged along the outer surface (16) of the insert. The projecting portion (13) comprises the contact ends (53, 54) of the one or more electrode and of the one or more counter-electrode, such that each electrode connects to a contact point (45) on the multi-well plate 2 and each counter-electrode connects to a contact point (35) on the lid (3), which is positioned on the plate (2) when the system is assembled.

## Description

### Technical domain

The present invention concerns instrumentation for manipulating and studying biological cell layers and tissues. In particular, the invention concerns a multi-well plate system for measuring cellular and trans-cellular impedance across one or more layers of a cell culture. This achieves the monitoring of cell behaviour (impedance) and overall tissue barrier function (trans epithelial electrical resistance).

### Related art

Filters and membranes are commonly used as a substrate to support and facilitate adherent cell growth, be it as a single layer or as multi-layered tissues. In medical and biotechnological *in vitro* models such membrane or filter supports are used to simulate biological barriers constituted of simple or complex mammalian and non-mammalian epithelia, and/or vegetal cells. The supports are often provided in well inserts which are suspended in a liquid, in particular a suitable growth medium, contained in individual wells of a multi-well plate. The chemical and physical parameters, as well as the inoculation with cells can be varied between individual wells and the effect of any these variations on the properties of the cells and/or the cell barrier can thus be determined.

Different methods can be employed to characterize the cells and measure the quality and/or the permeability of a cell layer or layers grown on such suspended supports. The easiest way to determine the integrity of the cell layer or layers is by simple observing the cells through a microscope. However, this method is often not sufficient in terms of accuracy and sensitivity. For this reason, microscopic observation is better suited as a redundant control method in support of more precise measurement methods.

Such measurement methods may be based on the ability of chemical compounds or ions to move through the cell layer, which may for example be determined electrochemically or through the use of labelled compounds.

The electrical resistance, and more generally the impedance across a cell monolayer or multiple layers of cells is a measure of its ion permeability and thus a very useful parameter to determine properties of the biological barrier, including its integrity. Ideally, the resistance should be determined non-invasively, such as not to inadvertently alter the properties of the biological barrier.

Impedance spectroscopy, using a number of defined frequencies, has proved particularly powerful for this purpose. Different from other electrochemical methods, which only work with direct current (DC) voltage or alternating current (AC) voltage with constant frequency, impedance spectroscopy uses AC voltage with sinusoidal, square or triangular waveform frequency, which varies during the measurement, such as to prevent interference when using small amplitudes. Impedance offers the advantage that further surface specific parameters, such as capacitance of the biological barrier, can be measured in addition to the electrical resistance. Spectroscopy measurements produce an impedance fingerprint based on the defined frequencies which are typical for cell culture type and biological barrier state and health,

WO2005098423 describes a multi-well assembly having a first tray with an array of sample wells and a second tray with a plurality of membrane systems. The second tray can be coupled with the first tray to form assay chambers with two compartments, which are separated by a cell layer across which ions can flow. Each sample well comprises an electrode with an electric connection passing thorough an opening in the wall of the well. The corresponding counter-electrodes protrude into the membrane systems from above. The properties of the cell layer are determined by measuring an electrical gradient across the layer grown on the membrane.

The system described in this document is based on electrodes and counter-electrodes, which are projecting into the liquid contained in the interior of each of the two chambers, which are separated by the membrane and the cell layer. In this arrangement there is a considerable risk that the electrodes contact or even damage the cell layer on the membrane. Moreover, the protruding electrodes are susceptible to damage themselves, as they can easily be bent or broken.

EP2322925A2 discloses a device to measure trans-cellular impedance of cells cultivated on a permeable membrane. The device comprises a first repository into which a second repository with a permeable membrane can be inserted. The device furthermore comprises a cover of the first repository. The cover has an electrode, which is contact with a conductive fluid contained in the second repository when it is positioned in the first repository and when the cover is positioned on the first repository. At least 50% of the inner surface of the first repository consists of conductive material. Similar to WO2005098423, the device described in this document relies on an electrode which protrudes into the liquid contained in the insert and is therefore positioned in closely to the cell layer. The risk of damages to the layer as well as to the electrode are therefore also significant disadvantages of this device.

Moreover, the electrodes and counter-electrodes of the systems described in these documents are each comprised in different components of the device. Hence, depending on the assembly of the device and the consistency in length of the electrodes, their position in respect of each other may vary slightly between the different sample wells or from plate to plate, potentially leading to an inconsistency in measurement.

Moreover, in systems in which an electrode is inserted into the well insert from the top, there remains a risk that the level of the liquid contained in the insert is not sufficiently high, such that the electrode does reach the liquid. In this case, no measurement can be performed. If liquid levels vary between the individual wells, this may lead to erroneous results.

### Short disclosure of the invention

An aim of the present invention is the provision of a multi-well plate system for measuring the properties of a single cell layer or a multi-layered biological tissue that overcomes the shortcomings and limitations of the state of the art. The system should be sensitive, accurate, non-invasive, traceable and robust to reproducibly determine the properties, in particular the integrity, of a biological barrier.

It is a further aim of this invention that the system should be easy to assemble and simple to manipulate. The risk of damaging the electrodes during handling of the different components of the system should be minimized.

According to the invention, these objectives are attained by the object of the appended claims, and especially by the independent claims.

The term layered cell culture as used herein includes monolayers of cells, as well as multi-layered cell cultures. Cells are preferably eukaryotic cells. Cells may however also be prokaryotic cells forming one or more layers, for example in the form of a biofilm. Preferably the cell layer is an epithelial monolayer.

Preferably the electrodes follow the surface and are not freely projecting into either the first or the second chamber, such that a bending or abrasion of the ends of the electrodes can be avoided. By arranging the electrodes along the surface of the insert, a damage to the cell barrier as a result of unintended movement or unsuitable dimension of the electrodes is also prevented.

Both electrodes are comprised in the well insert. The electrodes therefore are inserted into the well together with the insert as one unit. As a result, the positions of the electrodes in respect of each other are fixed. Their positions are also fixed in relation to the planar support of the insert, which gives rise to the barrier. This fixed arrangement permits for a rapid and dependable assembly of the sample wells with the two chambers and electrodes. It also results exact and reproducible positioning of the electrodes within the assembled sample wells, thus avoiding unintended variations between the wells.

Moreover, since the electrodes, and in particular the sensing portion of the electrodes are arranged flat along the surface of the insert, the electrodes are always at least partially submerged in the liquid (or conductive medium) when liquid (or conductive medium) is contained in the insert. The measurements are therefore not susceptible to different heights of liquid (or conductive medium) levels within the well insert.

The planar support is preferably a ultrafine or brittle membrane or a filter. It may be a permeable or semipermeable membrane. The membrane should be suited to facilitate the growth of cell culture layers. As mentioned above, such layered cell cultures may grow in form of monolayers, for example epithelial tissue monolayers. Alternatively, multi-layered biological tissues may be grown on the support. Multi-layered stands for stacked layers of cells of different types on the same side of the ultrafine membrane or a filter, realising complex co-culture cell-based systems, as opposed to mono-layered, which is a simplified one cell high cell layer. It is also thinkable that procaryotic cells are grown on the planar permeable or semipermeable support, for example for forming biofilms. The invention is not limited to the biological barrier formed on the planar support.

The insert should be suspended in the well such that the planar permeable or semipermeable support does not contact the inner surface of the well.

In a preferred aspect the first chamber is delimited by the insert wall and the planar support, and wherein the second chamber is delimited by the well and the outer surface of the insert.

The liquid contained in the first chamber, which is confined by the well, diffuses through the planar support into the second chamber, which is preferably entirely confined by the well insert. The liquid may be a suitable growth medium. The liquid may also be any other type of liquid composition. For example, the liquid may comprise substances affecting cell growth, such as growth factors or cytokines. The liquid may also comprise therapeutical drugs, toxic substances or detergents for testing the effect on such substances on the integrity of the biological barrier. Depending on the experimental objective, other types of compositions and/or substances may be added to the liquid.

In a preferred aspect, each insert has two separate pairs of electrodes suited for four-terminal measurement of electrical impedance. One electrode pair of electrodes may be used for carrying current while the other pair of electrodes may be used to sense voltage. Providing inserts with a four terminal measurement system as opposed to a two-electrode system increases the accuracy of the measurement.

Each of the electrodes may comprise an electrode lead connecting a sensing portion of the electrode, which is intended to be exposed to the conductive medium, with a contact end of the electrode for connecting to external electronic components.

The electrodes, respectively the sensing portions of the electrodes, may be positioned on a portion of the insert wall, which is submerged in the conductive medium when the sample well and insert are assembled for measuring.

The electrodes respectively the sensing portions of the electrodes may also be arranged on the inner and the outer surface of the planar support, provided the electrodes are not disposed on the permeable or the semi-permeable portion or portions of the planar support. Preferably, the sensing portions or sensing ends of the electrodes are not pointing away from the planar support. In other words, these end portions of the electrodes are preferably not sticking out into either the first or the second chamber of the assembled sample well. Ideally, the electrodes are positioned flat on the surface, closely following the surface of the insert. The electrodes do not rise significantly above the surface of the insert.

In one aspect of this invention, the electrodes follow at least partially the perimeter of the planar support. The electrodes may be arranged adjacent to the periphery of the at least permeable or semipermeable portion such that said one or more portion is at least partially surrounded by the electrodes. The electrodes are preferably disposed on the surface of the planar support, such that interference with cell growth and/or adhesion is avoided.

In a preferred embodiment, two electrodes are arranged around the periphery of the semi-permeable or permeable portion of the support in such a way that they form a loop, circle, or a circle with an opening. One electrode forms the inner loop or circle whereas the other electrode forms the outer loop or circle. In this embodiment, two counter-electrodes are attached on the outer surface of the planar support the same arrangement, one counter-electrode forming the inner loop or circle and the other counter-electrode forming the outer loop or circle. An opening in the loop or circle results from upward spatial arrangement along the insert wall of the electrode portion leading from the sensing portion to the contact end.

The invention furthermore concerns a multi-well device designed to receive the described well insert, comprising a plate with a plurality of wells for receiving well inserts, and a lid adapted to be disposed onto the plate.

In a preferred aspect the plate of the multi-well device has a plurality of first contact points for connecting to the contact end of the one or more, preferably two, first electrodes of each one or more well insert.

In a further preferred aspect, the lid of the multi-well device comprises a plurality of second contact points for connecting to the contact end of the one or more, preferably two, second electrodes of each one or more well insert. The second contact points comprised in the lid connect with the contact ends of the second electrodes of the one or more inserts when the lid is positioned on the plate.

The lid may comprise electrical connections to a printed circuit board for connecting to a processor. The printed circuit board may be attached to the lid. The PCB may also be integral part of the lid.

First, respectively the second, contact points are preferably arranged in such a way as to align and connect with the first, respectively the second, contact ends of the electrodes comprised in an insert, when the insert is disposed in the well and the lid is disposed on the plate.

To improve an accurate alignment of these electric contacts, the angular orientation of the inserts inside the well is preferably controlled by the shapes of the insert and the well. Such shapes may be matching in certain portions, for example a protrusion of the insert fitting into a corresponding recess in the well or the plate, or matching sectional shapes of at least a part of the insert and the well for engaging the insert in a defined angular position. Other suitable geometries are imaginable, the invention is not limited by the examples provided here. The angular position of the well may also be prescribed by additional alignment means, such as special clamps or separate stencil like alignment layers.

Inserts can be provided as individual entities for positioning into the wells. This may be useful when only a limited number of samples are tested in parallel. However, for the manipulation and screening of many samples in parallel, it is useful to provide the described inserts assembled in an array, for example an insert layer. This insert layer should be configured for positioning the inserts inside the wells of the multi-well plate. The insert may be removably attached to the insert layer. Alternatively, the insert may be integral parts of the insert layer.

The insert layer is preferably configured such that the contact ends of the first electrodes connect to the first contact points when the insert layer is positioned on the multi-well plate. Moreover, the contact ends of the second electrodes contained in the insert layer are preferably arranged to connect to the second contact points when the lid is positioned on the multi-well plate with the insert layer.

The multi-well plate system as described herein is particularly useful for culture of epithelial monolayers, wherein the cells are cultivated in the first chamber, preferably in form of a monolayer attached to the planar support, and the electrodes are arranged for measuring electrical impedance across the planar support. However, other suitable applications are possible.

As will be evident to the skilled person, the described multi-well system is readily adaptable for uses other than manipulation and growth of cell layers. The system having a semipermeable separation between the two chambers may for example also be used for determining physical and chemical parameter of adherent and/or suspended cell cultures. The multi-well system could also be adapted for chemical processes, measuring for example ion transfer or diffusion of substances across a membrane.

Electrodes could for example also be replaced by sensors, for example temperature sensors, which are attached to the surface of the insert in analogy to the electrodes and connected to external electronics by means of the contact points in the protrusion of the insert.

The invention places the electrodes remarkably close to the cell culture. This is especially advantageous when the cell culture produces or expresses physiological mucus or fluid products and may avoid or reduce the need of additional conductive liquid. An expert reader will readily appreciate the advantage of the invention with respect to the design of socalled "air-liquid" cell culture models

The present invention furthermore concerns a method of assembling the described multi-well plate system.

### Short description of the drawings

An exemplar embodiment of the invention is disclosed in the description and illustrated by the drawings in which:
**Figure 1a and 1b** illustrate schematically a perspective view of an exemplar embodiment of the well insert, view from above, respectively from below.
**Figure 2a and 2b** show a multi-well plate in perspective, from above, respectively from below.
**Figure 2c** shows an indexing layer
**Figure 3a and 3b** show a lid for the multi-well plate in perspective, from above, respectively from below.
**Figure 4** schematically illustrates a cross section through a well with a well insert of the exemplary embodiment.
**Figures 5a-5c** are details of the upper connector (A) of figure **4****,** in three variants.
**Figures 6a-6e** are details of the lower electrodes (B) of figure 4, in five variants.

### Examples of embodiments of the present invention

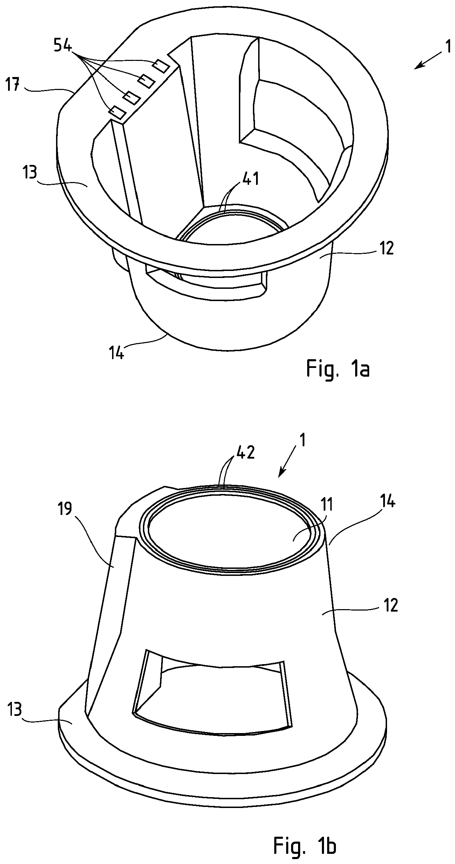

Figure 1a shows a possible embodiment of a well insert 1. The insert 1 has an upper portion with a projecting element or flange 13 that extends outwards and is configured to interface with a multi-well plate such that the insert 1 can be suspended in one of the wells of the plate, as it will be disclosed later. The flange has positioning means that interact with corresponding features of the multi-well plate in such a way that the position of the inserts suspended in the wells are completely determined without the possibility of turning, wobbling or tilting. Preferably, the link between the insert and the multi-well plate is easily reversible, and the inserts can be extracted and replaced easily from the wells.

In this example, the positioning feature is shaped like a flat ring flange encircling the insert, and the positioning means include the circular rim of the flange 13 and the straight section 17. These positioning means are designed to fit in recesses having the corresponding shape in the multi-well plate. This is not the only configuration possible, for example a plurality of distinct projecting portions protruding from the insert walls.

The lower portion of the insert 1 has a bottom 14 connected to the upper section by a side wall 12. The bottom is represented as a flat disc: a shape that is practically advantageous but not essential.

When the insert is suspended in a well the bottom portion 14 is lifted above the bottom of the well without touching it and the side wall 12 is separated from the inner surfaces of the well 20. Preferably, the wall 12 of the insert is inclined outwards towards the upper portion 13. The insert is stably suspended in the well of the plate without contacting the interior surface area of the well.

Figure 1b shows the same insert from below. The bottom comprises a semipermeable or permeable portion 11 that is suitable for supporting a cell culture, preferably a filter membrane or a brittle membrane. Growth of cells, respectively the formation of a cell layer, on the semi-permeable or permeable portion 11 alters the electric properties of the barrier. The impedance across this biological barrier formed by the cell layer increases with its integrity. In other words, a substantially imperforate cell layer preventing the transfer of ions between the chambers will result in an increased impedance, while a layer permitting leakage of ions between the chambers has a lower impedance. The electric impedance across the cell culture is an important biological parameter.

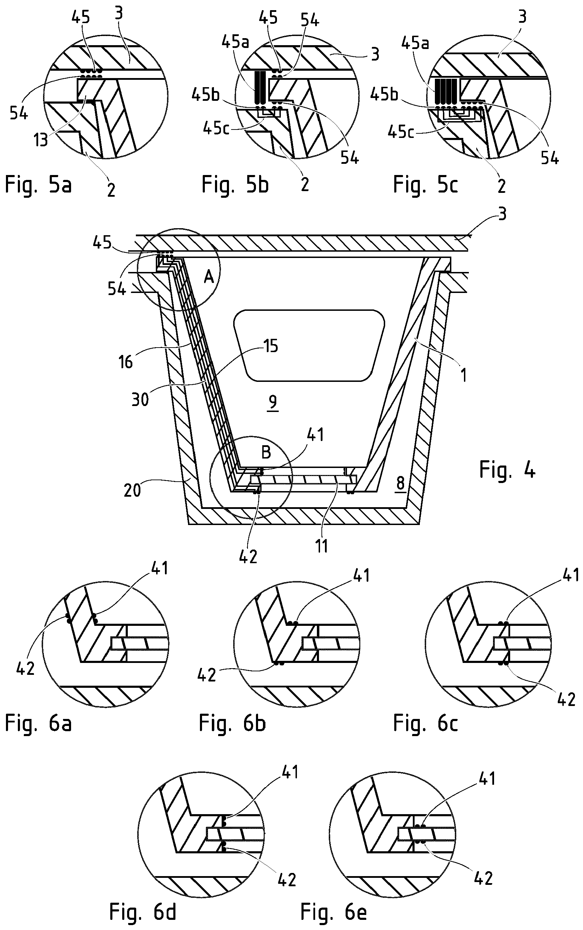

The insert 1 is inserted into the well 20 such as to separate two chambers, as is shown in Figure 4. In the depicted embodiment an outer chamber 8 is confined by the interior surface of the well 20 and the outer surface 16 of the insert. The inner chamber 9 is delimited by the inner surface 15 of the insert. As shown in this example, the lower part 14 of the insert suspended in the well provides a barrier between the first and the second chamber and holds the semi-permeable or permeable portion 11.

The assembled sample wells 20 with the two chambers 8, 9 defined by the well insert 1 inserted therein, can receive a growth medium or any other liquid suitable for the growth and/or the manipulation of the cell culture on the membrane 11. The liquid may be a growth medium. The liquid may also be any other liquid, optionally comprising compositions or substances chosen for a particular purpose. The liquid is contained in the well and diffuses into the inserted insert, such that the electrodes 41, 42 are at least partially submerged in the liquid to perform the measurements. At least a sensing portion of the electrodes must be submerged in the liquid to enable impedance measurement.

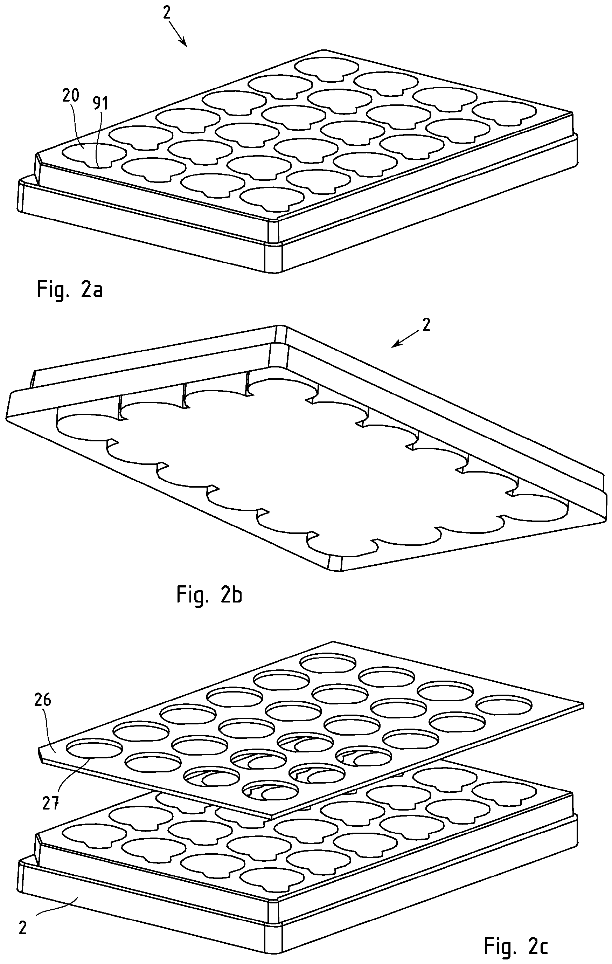

Figures 2a and 2b show a possible realization of a multi-well plate. Each plate comprises a plurality of wells 20, each configured to receive an insert. The multi-plate wells may be made of biocompatible plastic.

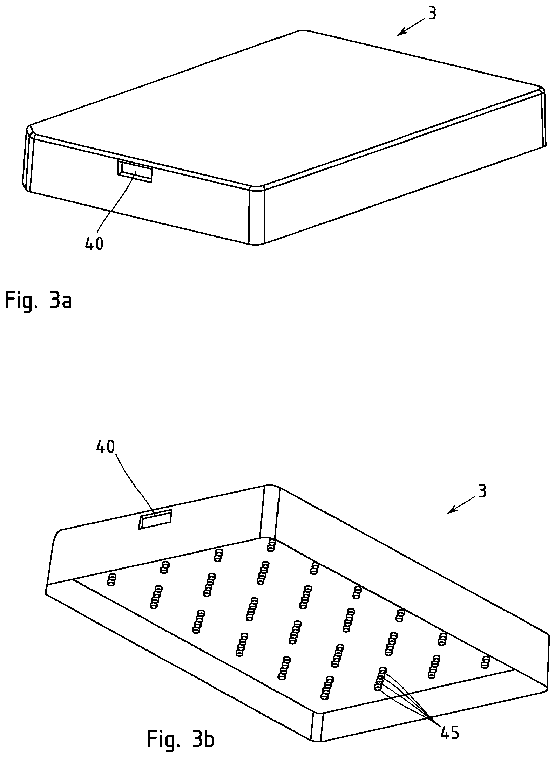

The multi-well plates are covered by lids, as shown in figures 3a and 3b. The lids, conventionally, prevent evaporation and allow the stacking of many multi-well plates in an environmental chamber. In the invention, the lids have the additional function of collecting electrical impedance signals through the contacts 45, as it will be disclosed later. Each well position is equipped with a plurality of contact points 45 in the lid, preferably at least four contact points per well.

The inserts 1 comprise one, preferably two pairs of the electrodes in the lower portion 14. Each pair has an inner electrode 41 in the inner chamber 9 and a counter electrode 42 in the outer chamber 8. The electrodes are exposed for realizing a galvanic contact with the liquid in the inner and outer chambers. They can be used to measure an impedance of the cell culture growing on the membrane 11. A system of four electrodes in two pairs can be used to carry out a four-terminal impedance measurement, also known as a Kelvin sensing, which is preferable because it is not affected by spurious wire impedances, which affect two or three-terminal based methods. In alternative variants, the electrodes can be configured to measure other parameters, such as difference in electrochemical potential, or difference in oxygen levels, or temperature, or pH/pOH, or provide thermoelectric heating or cooling.

In the embodiment depicted in Figures 1a and 1b, two first electrodes 41 are attached to the inner surface of the insert, on the planar support or close thereto, while two corresponding counter-electrodes 42 are on the outer side of the insert 1, which is facing the outer chamber. The first electrodes 41 and the second electrodes are represented as concentric loops or circles. This is not an essential feature, however.

The inner and outer electrodes 41, respectively 42 are in electric connection with contact pads 54 on the upper portion of the insert 1. The connection can be obtained in several manners. In a preferred variant, however, the electrodes are connected each with a corresponding contact pad by a conductor buried under the surface of the insert. The tracks may run approximatively straight along the side wall 12 or follow any suitable path. In the represented variant, the contact pads face upwards, but other dispositions will be disclosed later.

In a preferred variant, the insert 1 is a moulded interconnect device on which electrodes 41, 42, the contact pads 54 and the conductors that join them are integrally fabricated. In a possible variant, a core part of the insert is moulded using a resin compound that can be activated by means of a laser irradiation, for example ABS, or a polymer doped with a non-conductive laser-activable metallic compound. Then the desired electrodes 41, 42, pads 54 and connecting tracks are written on the inner and outer sides of the core with a laser, and then the parts activated by the laser are covered by metallic track in a metallization step, for example in an electrodeless copper bath, whereupon the desired conductor electrodes, pads and track arise precisely where the plastic has been activated by the laser.

The conductive tracks can be completed by successive layers of copper, nickel or gold, either in electrodeless or electrolytic processes, and preferably by a top finish layer of a bioinert metal, for example gold.

Other processes for the realization of moulded interconnected devices foresee the writing of the electrode, pads and tracks in conductive ink, for example by inkjet, or screening, and successive metallization as above. They are also possible in the scope of the invention.

The insert is completed by a biocompatible layer on both sides. The biocompatible layer covers entirely the resin compound of the core and the tracks and has apertures above the contact pads 54 and the electrodes 41, 42.

The biocompatible layer may be a polycarbonate film or a conformal layer applied in any suitable process including, but not limited to spraying, brushing, dip coating, over moulding, or deposed from a gas phase.

Figures 2A and 2B show an exemplary embodiment of a multi-well plate 2. The insert depicted in Figures 1a and 1b can be positioned in the wells 20 of the plate. Individual inserts 1 can be placed into individual wells 20, as shown in Figure 4. Preferably, the plate 2 holds a plurality of identical wells 20 in a regular array, but the invention does not exclude realization in which one or some of the wells may be special and different from others. In non-represented variants two or more inserts can be combined in one piece capable of fitting in multiple wells 20 of the plate 2.

Figures 3A and 3B show an exemplary lid 3 of the multi-well plate device, which is positioned on top of the multi-well plate 2 when the multi-well device is assembled. In correspondence with each well 20, there is a connector provided on the inside of the lid 3. Each connector comprises a plurality of contact points 45 which are precisely positioned at the points where the contact pad 45 of each insert placed in the wells lie. In this way, the electrodes of each insert are automatically connected with the lid 3 when the multi-well system is assembled.

Preferably, the contact points 45 are arranged on a printed circuit board (PCB) inserted in the lid 3, for example be attached to or integral with the central plate of the lid. The printed circuit board carries conductive tracks to route the electrical signals obtained by the contact points 45 to an external processing system, for example a multichannel impedance analyser configured to determine the impedance across the cell cultures in the inserts. The printed circuit may as well include active components, for example amplifiers, filters, digitizers, and multiplexers, that are configured to carry out the processing fully or party in the lid itself. Opening 40 are used to lead out electric cables or wires for connecting the external processing system. They may be replaced, in variants, by suitable connectors.

The correct alignment of the insert 1 in the well 20 ensures that, when the lid 3 is closed on the well plate 2, each of the pads 45 is touched by one contact respective contact points 45 on the lid 3. The alignment is ensured by suitable indexing features on the insert 1 matching a corresponding feature of the well 20, such that the inserts 1 can be suspended in the wells 20 in a predetermined position and orientation. There are many ways to achieve this result, and all are comprised in the invention. In the represented example, the position is ensured by the protrusion 19 on the outside surface of the side wall 12 and/or the straight segment 17 of the flange 13 (see figure 1), which engage in the recess 91 of the well 20 and in a non-represented ridge on the top flat face of the multi well plate 2.

In the variant shown in figure 2c the alignment and positioning of the wells is ensured by a separate alignment layer 26 that is configured to sit on a predetermined position on the top face of the plate well 2 and has features that determine univocally the position and the orientation of the inserts 1, for example the straight edges 27 that are configured to interact with the corresponding straight segments 17 of the flanges of the inserts (see figure 1). Alternatively, as mentioned above, the inserts can be provided as part of an insert array, in which several inserts are united in a rigid regular grid matching the disposition of the wells in the plate 2, such that their position and orientation are implicitly determined.

The disposition of electrodes and contact pads disclosed so far can be modified and adapted considerably without leaving the true scope of the invention. The electrodes 41, 42 can have any shape and, while it is advantageous to have them encircling closely the membrane 11 where the cell culture grows, they can be disposed in many ways. Figure 4 shows an insert 1 in a well 20, in cross section, and has the electrodes 41 on a vertical wall that limits the membrane 11, while the external electrodes 42 are on a lower face of the insert 1, facing the bottom of the well 20.

Figures 6a-6e show the detail 'B' of figure 4 and illustrate alternative positions of the electrodes 41 and 42 either on vertical walls limiting the membrane, or on the horizontal bottom of the insert, or on the lowermost part of the inclined wall. All are suitable for realizing the invention.

The position of the contact pad in the upper portion of the insert 1 allows several variants, in the frame of the invention. Figures 5a-5c show the detail 'A' of figure 4, in three possible variants. In figure 5a all the contact pads 54 are on an upper side of the flange 13, as in figure 1, and the contact points 45 reach them from the inner side of lid 3.

In the variant of figure 5b two out of the four pads 54 are on the upper side of the flange and remaining two pads 54 are on a lower opposite side of the flange. The two pads 54 are reached by two contact points 45 as in the previous disposition. The contact to the lower pads 54 is made through contact points 45a that reach down to an upper face of the multiwell plate 2 where they touch contact pads 45b which are connected to the pads 45a on the insert through conductors 45c.

In the variants of figure 5c all the contact pads 54 lie on the lower side of the flange 13 and are connected to the lid through conductors 45c, pads 45b on the upper face of the multi well plate 2 and contact points 45a.

### Reference symbols

- 1: insert
- 2: multi well plate
- 3: lid
- 8: outer chamber
- 9: inner chamber
- 11: membrane
- 12: wall
- 13: flange, protruding upper section
- 14: bottom, lower section
- 15: inner surface of the insert
- 16: outer surface of the insert
- 17: straight segment of the flange
- 19: protrusion
- 20: well
- 26: alignment layer
- 27: straight segment of the alignment layer
- 30: conductive tracks
- 40: opening or connector
- 41: inner electrodes
- 42: outer electrodes
- 45: contact points
- 45a: contact points
- 45b: contact pads
- 45c: conductors
- 54: contact pads on the insert
- 91: recess

## Claims

1. A well insert (1) for a multi-well plate (2) for use in cell culture having an upper portion (13) interfaceable with the multi-well plate (2) to suspend the insert (1) in a position in a well (20) of the multi-well plate, a lower portion (14) positioned in the well below the upper portion when the insert is in the predetermined position and comprising an aperture, a permeable or semi-permeable portion (11) for supporting a cell culture closing the aperture, a wall (16) joining the upper portion to the lower portion, wherein the insert in position partitions the well volume into an inner chamber (9) and an outer chamber (8), **characterized in that** the lower portion (14) has inner electrodes (41) and outer electrodes (42), respectively in the inner chamber and in the outer chamber, each electrode being connected to a corresponding contact pad (54) on the upper portion (13) by an electrically conductive element (30), wherein the position of the contact pads (54) is completely defined when the insert (1) is in position.

2. The well insert of claim 1, comprising two inner electrodes and two outer electrodes for four-point measuring of electrical impedance across the cell culture.

3. The well insert of any of the preceding claims, the electrically conductive elements (30) being metal traces plated on a moulded thermoplastic material.

4. The well insert of the preceding claim, comprising a core of a thermoplastic polymer on which the metal traces are plated and a protective layer of a biocompatible polymer covering the metal traces.

5. The well insert of the preceding claim, wherein the protective layer is transparent.

6. The well insert of claim 4 or 5, wherein the thermoplastic polymer is any one of ABS, or a polymer doped with a non-conductive laser-activable metallic compound, and/or the protective layer is polycarbonate.

7. A multi-well device designed to receive the well insert (1) of any one of the preceding claims 1 to 6 comprising a multi-well plate (2) having a plurality of wells interfaceable with the insert (1) to suspend the insert (1) in a position in a well (20) of the multi-well plate (2), a lid (3) for covering the multi-well plate (2) and contact points (45) in the lid and/or in the multi-well plate facing the positions of the contact pads of the insert.

8. The multi-well device of the preceding claim, comprising a printed circuit board (PCB) in the lid (3) carrying electronic components for acquiring and/or processing and/or concentrating signals related to the impedance of the cell culture.

9. The multi-well device of the preceding claim, wherein the lid (3) further comprises a connector for a data bus.

10. The multi-well device of any one of claims 7-9, comprising an aligning mask with positioning means interfacing with the multi-well plate and with the inserts to determine the position of the insert and of the contact pads when the insert is suspended in a well.

11. Use of a multi-well plate system comprising culturing cells on the permeable or semi-permeable portion of the well insert of any one of claims 1 to 6, connecting an impedance-measuring device to the contact pads and measuring an electrical impedance of the cell culture through the electrodes.

12. Method of manufacturing an instrumented well insert according to any one of claims 1-6 comprising:
- forming a substrate of a first thermoplastic polymer with an upper part, a lower part and a connecting element joining the upper part and the lower part,
- printing, plating, or depositing metal on the substrate forming one or more pairs of electrodes on the lower part of the substrate, each electrode being connected to a corresponding contact pad on the upper part of the substrate by a metal trace running along the connecting element,
- covering the substrate with a second thermoplastic polymer that is biocompatible.

13. The method of the preceding claim, wherein the electrodes, traces and pads are realized with a LDS (Laser Direct Structuring) process.
